# EUROPEAN PATENT SPECIFICATION

(11) **EP 4 142 680 B1**
(45) Date of publication and mention of the grant of the patent: **18.10.2023**
(21) Application number: 21717903.5
(22) Date of filing: 14.04.2021
(51) Int. Cl.: A61K 8/34, A61K 8/44, A61Q 19/00

(54) **STABILIZED COSMETIC COMPOSITIONS WITH N, N'-DI-ACETYL CYSTINE**
STABILISIERTE KOSMETISCHE ZUSAMMENSETZUNGEN MIT N,N'-DIACETYL-CYSTINE
COMPOSITIONS COSMÉTIQUES STABILISÉES COMPRENANT DE LA CYSTINE DE N, N'-DIACETYLE

(30) Priority: 28.04.2020 EP 20171853
(43) Date of publication of application: 08.03.2023
(73) Proprietor: Unilever IP Holdings B.V., 3013 AL Rotterdam (NL); Unilever Global IP Limited, Wirral, Merseyside CH62 4ZD (GB)
(72) Inventor: ROSA, Jose, Guillermo, Trumbull, CT Connecticut 06611 (US); MOADDEL, Teanoosh, Trumbull, CT Connecticut 06611 (US)
(74) Representative: James, Helen Sarah
(86) International application number: PCT/EP2021/059653
(87) International publication number: WO 2021/219378

(56) References cited:
- EP-A1- 1 374 831
- WO-A1-2018/114745
- US-A- 4 827 016
- US-A1- 2008 305 059

## Description

### Field of the invention

Provided herein are topical cosmetic compositions containing 4-substituted resorcinol derivatives which are stabilized with N,N'-diacetyl-L-Cystine (NDAC).

### Background of the invention

Aging skin is influenced by numerous physiological and genetic factors. Ideal skin should be smooth and even, with no apparent surface flaws and with uniform color distribution.

Resorcinol (1,3-benzenediol) derivatives have been used to provide cosmetic benefits to hair and skin, such as skin lightening, as well as other cosmetic uses. 4-Substituted resorcinol derivatives and their preparation have been described, see, for example, U.S. Patent No. 4,959,393; U.S. Patent No. 6,132,740; U.S. Patent Application Publication Nos. 2004/0042983 and 2008/0131382; Japanese Published Patent Application Nos. JP 2001-01 0925 and JP 2000-327557. However, some of these compounds can be difficult to formulate, as they are unstable in a cosmetically acceptable vehicle or in cosmetic compositions. 4-Substituted resorcinol derivatives are susceptible to oxidative and/or color instability, exemplified by darkening of compositions in which they are contained.

There is a need, therefore, for an agent that will stabilize 4-substituted resorcinols against oxidation and/or discoloration. In particular, there is a need for an agent that will prevent the oxidation and/or discoloration of 4-substituted resorcinols and compositions which contain them. Furthermore, there is a need to have topical cosmetic compositions that contain resorcinol derivatives that are stabilized against factors such as discoloration and/or oxidation, and which exhibit general storage stability.

For additional cosmetic skin benefits, niacinamide, which is the amide form of vitamin B3 (Niacin), may be included in topical compositions. Vitamin B3 is an essential water-soluble vitamin and is found in the form of niacin or niacinamide in a wide variety of food including meat, fish, legumes, nuts, grains, mushrooms, yeast and coffee. It is implicated in various cellular functions like DNA repair, keratinocyte differentiation, lipid synthesis, pigmentation and inflammation, most important of them being in cellular bioenergetics. Niacinamide in the form of pyridine nucleotide, nicotinamide adenine dinucleotide (NAD), is important for energy metabolism. A diet deficient in B3 leads to disease called Pellagra characterized by skin conditions with dermatitis and pigmentation. This was the observation that had led to the prior discovery of niacinamide as a skin lightening agent.

Like vitamin B3, cystine is normally derived from the diet and has cosmetic benefits. Delivery of cystine from topical compositions, however, is challenging due to its extremely low solubility in biologically acceptable vehicle in a neutral pH range, which is the pH range required for topical application. The solubility of cystine in water is low as it tends to crystallize out of solution. N-acetyl-L-cysteine ("NACys") inhibition of cystine crystal growth is disclosed in Cryst. Eng. Comm, 2016, 18, 8587.

Compositions for potentiating intracellular glutathione production have been described. See e.g. Chiba et al. US Patent 7,740,831, Crum et al (USRE37934, USRE42645, WO2016/033183, and US20050271726); Mammone US Patent 6,149,925, and Perricone US 20060063718. Topical compositions and enhancing generation of glutathione in skin from its constituent amino acids (glutamate, cysteine, and/or glycine, i.e., glutathione precursors) for cellular uptake and synthesis of the GSH tripeptide was addressed in, e.g., Applicant's U.S. Published Patent Application Nos.: US20/9034, US20/16059, and US19/328631.

A number of cysteine/cystine derivatives, including b-substituted cysteine/cystines, cystine diamides, cystine dialkyl esters, N-alkanoylcysteines (such as "NACys") and N,N'-dialkanoylcystines (such as N,N'-diacetyl-L-cystine or "NDAC") have been described in connection with organ benefits for kidneys, such as prevention of cystine stone formation. NDAC, among other compounds, was also studied for Cystine crystal growth inhibition, although it did not work. See, Cryst. Eng. Comm, 2016, 18, 8587; US2014/187,546 (Rutgers State Univ Of New Jersey); Zhu, et al., "Rational Design of Novel Crystal Growth Inhibitors for Treatment of Cystinuria Kidney Stones," 2013

### ProQuest Dissertations and Theses.

Skin cosmetic compositions using NDAC and/or NDAC salt have not been found.

### Brief Description

The compositions comprising 4-alkyl resorcinols stabilized with N,N'-Diacetyl-L-Cystine ("NDAC") described herein obviate the deficiencies in the prior art described above.

Provided herein is a topical cosmetic composition comprising:
a. 0.0001 to 10 wt % of N,N'-diacetyl-L-Cystine; preferably less than 1 to 2wt% of N,N'-diacetyl-L-Cystine; more preferably 0.2wt% of N,N'-diacetyl-L-Cystine;
b. 0.00001 to 10 wt% of one or more 4-substituted resorcinol derivatives of the general formula I; preferably 1-2wt% of a 4-substituted resorcinol derivative of the general formula I; more preferably 1 wt % or less of a 4-substituted resorcinol derivative of the general formula I; alternatively 0.4wt% of a 4-substituted resorcinol derivative of the general formula I: 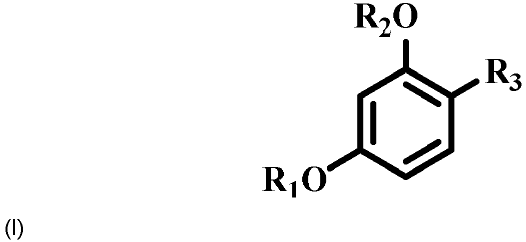
   wherein each R₁ and R₂ independently is selected from the group consisting of a hydrogen atom, -CO-R, -COO-R_{,} and CONHR; wherein R represents a saturated or unsaturated, linear, branched or cyclic C₁ - C₁₈ hydrocarbon;
   wherein R₃ represents a saturated or unsaturated, linear branched or cyclic C₁ - C₁₈ hydrocarbon;
      and
c. a cosmetically acceptable vehicle.

The 4-substituted resorcinol derivative is stabilized against degradation/discoloration/oxidation with inclusion of NDAC in the composition. The compositions comprise cosmetically effective amounts of the resorcinol compounds and NDAC in an amount sufficient to stabilize the resorcinol compounds against chemical degradation as evidenced by discoloration.

The resorcinol derivatives provided herein, which are defined below, are useful in the treatment or prevention of one or more cosmetic conditions as desired by the subject in need thereof, such as to prevent, lighten, reduce or treat the signs or appearance of undesired pigmentation of skin affected by the one or more conditions.

The 4-substituted resorcinol derivatives have the general formula I as defined above (I)
and preferably include 4-linear alkyl resorcinols, 4-branched alkyl resorcinols, 4-cycloalkyl resorcinols, and mixtures thereof.

More particularly preferred resorcinol derivatives include 4-ethyl resorcinol, 4-isopropyl resorcinol, 4-hexyl resorcinol and 4-cyclohexyl resorcinol, as well as O-acylated versions thereof. Most preferred resorcinol derivatives are 4-ethyl resorcinol and 4-hexyl resorcinol.

In another embodiment, provided is a stabilized cosmetic composition comprising:
a. 0.1 to 2% of a 4-substituted resorcinol derivative;
b. about 0.0001 wt % to about 5 wt % of N,N'-diacetyl-L-Cystine;
c. an optional skin benefit agent; and
d. a cosmetically acceptable vehicle;

wherein the weight ratio of said N,N'-diacetyl-L-Cystine to said 4-substituted resorcinol derivative is about 10,000:1 to about 1:100,000; and
wherein said NDAC stabilizes said 4-substituted resorcinol derivative, or said optional skin benefit agent, or both, in said vehicle.

The inventive compositions are aesthetically pleasing and the resorcinol derivatives therein have improved storage/color/oxidative stability.

Also provided is a method of stabilizing (against oxidation, discoloration, storage, etc) 4-hexyl resorcinol in a composition comprising a cosmetically acceptable vehicle by adding to the composition NDAC in an amount sufficient to stabilize said 4-hexyl resorcinol.

Also provided is a method of using N,N'-diacetyl- L-Cystine and 4-substituted resorcinol derivatives in the manufacture of a cosmetic composition for topical application to the skin of an individual.

In another variation, provided herein is a method of regulating a skin condition by topically applying an effective amount of the inventive compositions comprising 4-substituted resorcinol derivatives and N,N'-diacetyl-L-Cystine to the skin of an individual in need thereof.

Also provided herein is a product comprising instructions directing a user to apply a composition including a skin care composition comprising one or more 4-substituted resorcinol derivatives, which compositions also contain NDAC as a stabilizer. The composition can comprise pharmaceutically and/or dermatologically acceptable carriers and vehicles.

The inventive compositions are aesthetically pleasing and have improved storage/oxidative stability.

### Detailed Description of the Invention

### Definitions

As used herein, "administering to skin in need of such treatment" means contacting (e.g., by use of the hands or an applicator such as, but not limited to, a wipe, tube, roller, spray, or patch) the area of skin in need such treatment or an area of skin proximate to the area of skin in need of such treatment.

As used herein, "composition" means a composition suitable for topical administration to the skin.

As used herein, the term "cosmetic composition" is intended to describe compositions for topical application to human skin, including leave-on and wash-off products.

The term "skin" as used herein includes the skin on the face, neck, chest, back, arms, axillae, hands, legs, and scalp of a human.

Except in the examples, or where otherwise explicitly indicated, all numbers in this description indicating amounts of material or conditions of reaction, physical properties of materials and/or use are to be understood as modified by the word "about". All amounts are by weight of the composition, unless otherwise specified.

For the avoidance of doubt the word "comprising" is intended to mean including but not necessarily consisting of or composed of. In other words the listed steps or options need not be exhaustive.

For all compositions described herein, and all methods using a composition described herein, the compositions can either comprise the listed components or steps, or can "consist essentially of" the listed components or steps. When a composition is described as "consisting essentially of" the listed components, the composition contains the components listed, and may contain other components which do not substantially affect the skin or the skin condition being treated, but do not contain any other components which substantially affect the skin or the skin condition being treated other than those components expressly listed; or, if the composition does contain extra components other than those listed which substantially affect the skin or the skin condition being treated, the composition does not contain a sufficient concentration or amount of the extra components to substantially affect the skin or the skin condition being treated. When a method is described as "consisting essentially of" the listed steps, the method contains the steps listed, and may contain other steps that do not substantially affect the skin or the skin condition being treated, but the method does not contain any other steps which substantially affect the skin or the skin condition being treated other than those steps expressly listed.

As used herein, the term "cosmetically-acceptable" means that the compositions or components thereof so-described are suitable for use in contact with skin, particularly human skin, without undue toxicity, incompatibility, instability, irritation, or allergic response.

As used herein, the term "cosmetically acceptable carrier" or "cosmetically acceptable vehicle" includes all solvents, dispersion media, coatings, antibacterial and antifungal agents, isotonic and absorption delaying agents and the like, that are cosmetically acceptable. Except insofar as any conventional media or agent is incompatible with the active ingredient, its use in the cosmetic compositions is contemplated. Supplementary active ingredients can also be incorporated into the compositions. Cosmetically acceptable carriers are suitable for topical application to the skin, have good aesthetic properties, are compatible with the active agents described herein and any other components, and cause minimal or no safety or toxicity concerns. A safe and effective amount of carrier is from about 50% to about 99.99% or about 50% to about 99%, preferably from about 80% to about 99.9% or about 75% to about 99%, more preferably from about 90% to about 98%, and most preferably from about 90% to about 95% or about 85% to about 95% of the composition. The percentages are preferably percent by weight.

As used herein, the term "topical application" means to apply or spread the compositions described herein onto the surface of the skin.

As used herein, the term "4-substituted resorcinol" are defined hereinbelow with reference to Compounds of General Formula I.

As used herein, "alkyl" is intended to embrace a saturated linear or branched hydrocarbon chain having the number of carbon atoms specified. In one embodiment, alkyl groups can have 1 to 12 carbon atoms. "Alkylene" is intended to embrace a divalent saturated linear or branched hydrocarbon chain having the number of carbon atoms specified. In one embodiment, alkylene groups can have 1 to 12 carbon atoms.

As used herein, "cycloalkyl" is intended to embrace a saturated cyclic hydrocarbon chain having the number of carbon atoms specified. In one embodiment, cycloalkyl groups can have 3 to 12 carbon atoms.

As used herein, "alkenyl" is intended to embrace a linear or branched hydrocarbon chain having at least one carbon-carbon double bond. In one embodiment, alkenyl groups can have 2 to 12 carbon atoms.

As used herein, "aryl" is intended to embrace an aromatic hydrocarbon such as for example a substituted or unsubstituted phenyl or naphthyl group.

As used herein, the term "stabilized" means reducing discoloration and odor that would otherwise appear during storage of a compositions for any period of time.

While the compounds described herein can occur and can be used as the neutral (non-salt) compound, the description is intended to embrace all salts of the compounds described herein, as well as methods of using such salts of the compounds. In one embodiment, the salts of the compounds comprise pharmaceutically acceptable salts and/or dermatologically acceptable salts. Cosmetically acceptable salts are those salts which can be applied to the skin of humans and/or animals and which, upon application, retain at least some of the biological activity of the free compound (neutral compound or non-salt compound). The desired salt of a basic compound may be prepared by methods known to those of skill in the art by treating the compound with an acid. Examples of inorganic acids include, but are not limited to, hydrochloric acid, hydrobromic acid, sulfuric acid, nitric acid, and phosphoric acid. Examples of organic acids include, but are not limited to, formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, sulfonic acids, and salicylic acid. Salts of basic compounds with amino acids, such as aspartate salts and glutamate salts, can also be prepared. Examples of inorganic salts of acid compounds include, but are not limited to, alkali metal and alkaline earth salts, such as sodium salts, potassium salts, magnesium salts, and calcium salts; ammonium salts; and aluminum salts. Examples of organic salts of acid compounds include, but are not limited to, procaine, dibenzylamine, N-ethylpiperidine, N,N-dibenzylethylenediamine, triethylamine or trialkylamine salts. Salts of acidic compounds with amino acids, such as lysine salts, can also be prepared.

### Compositions

The topical cosmetic compositions according to the present invention include but are not limited to components described hereinbelow.

Provided herein is a topical cosmetic composition comprising:
a. about 0.0001 to about 10 wt% of NDAC, preferably less than 1-2 wt% NDAC; more preferably 0.2 wt% NDAC;
b. about 0.00001 to about 10 wt % of a 4- substituted resorcinol derivative, preferably 1-2 wt% of a 4-substituted resorcinol derivative ; more preferably 1 wt % or less of a 4-substituted resorcinol derivative; alternatively 0.4 wt% of a 4-substituted resorcinol derivative;
   and
c. a vehicle or carrier.

### 4-Substituted Resorcinol Derivatives

Resorcinol derivatives are known compounds and can be readily obtained, for example, by a method wherein a saturated carboxylic acid and resorcinol are condensed in the presence of zinc chloride and the resultant condensate is reduced with zinc amalgam/hydrochloric acid (Lille. J. Bitter, LA. Peiner. V, Tr. Nauch-Issled. Inst. slantsev 1969, No. 18, 127), or by a method wherein resorcinol and a corresponding alkyl alcohol are reacted in the presence of an alumina catalyst at a high temperature of from 200 to 400° C (British Patent No. 1,581,428). 4-alkyl resorcinols are commercially available from Sigma-Aldrich, St. Louis, Missouri, USA.

The inventive compositions comprise as an effective amount of a resorcinol derivative of the following formula (I): 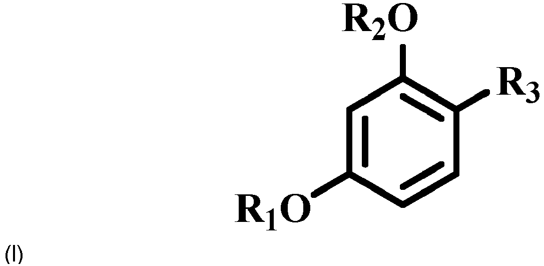 Each R₁ and R₂, independently, represents a hydrogen atom, -CO-R (acyl group), -COO-R_{,} CONHR; the latter three represented by the following formula A, respectively: 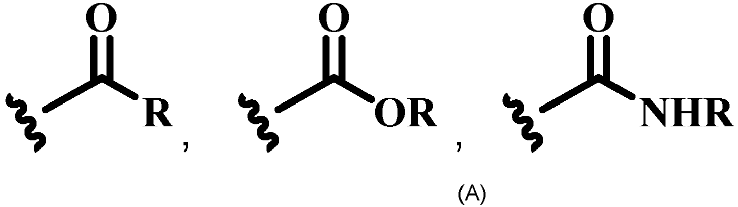 where R represents saturated or unsaturated, linear branched or cyclic C₁ - C₁₈ hydrocarbon. In a preferred embodiment, each or both R₁ and/or R₂ represents hydrogen. In a more preferred embodiment, both R₁ and R₂ represent hydrogen.
R₃ represents: a saturated or unsaturated, linear branched or cyclic C₁ - C₁₈ hydrocarbon, preferably a C₂ - C₁₂ hydrocarbon.

### 4- Substituted Resorcinols: Linear or Branched

In the above formula (1), the group represented by R₃ and preferably having from 2 to 12 carbon atoms, where the arrangement is linear, may include an ethyl group, a propyl group, a butyl group, a pentyl group, a hexyl group, a heptyl group, an octyl group, a nonyl group, a decyl group, an undecyl group and a dodecyl group. These linear alkyl groups may be substituted with a methyl or ethyl group at one or more hydrogen atoms thereof. Specific examples of the substituted alkyl group include an isopropyl group, an isobutyl group, an isoamyl group, a 2-methylhexyl group and the like. Preferred alkyl groups are those where R₃ is an ethyl, propyl, isopropyl, butyl, pentyl, hexyl, heptyl, or octyl group. The most preferable alkyl resorcinols are those where R₃ is an ethyl, isopropyl, or hexyl group.

Resorcinol derivatives of formula (I) are preferably the compounds selected from the group consisting of 4-ethylresorcinol, 4-propylresorcinol, 4-isopropylresorcinol, 4-butyl resorcinol, 4-pentylresorcinol, 4-hexylresorcinol, 4-heptylresorcinol, 4-octylresorcinol, 4-nonylresorcinol, 4-decylresorcinol, 4-dodecylresorcinol, 4-(1-methylpropyl)resorcinol, 4-(1-ethylpropyl)resorcinol, 4-(1-phenylethyl)resorcinol, 4-(2-phenylethyl)resorcinol, 4-(1-phenylpropyl)resorcinol, 4-(2-phenylpropyl)resorcinol, 4-(3-phenylpropyl)resorcinol, 4-(1-phenylbutyl)resorcinol, 4-(2-phenylbutyl)resorcinol, 4-(3-phenylbutyl)resorcinol, 4-(4-phenylbutyl)resorcinol, 4-(1-phenylhexyl)resorcinol, 4-(2-phenylhexyl)resorcinol, 4-[1-(2-methylphenyl)ethyl]resorcinol, 4-[1-(3-methylphenyl)ethyl]resorcinol, 4-[1-(4-methylphenyl)ethyl]resorcinol, 4-[2-(2-methylphenyl)ethyl]resorcinol, 4-[2-(3-methylphenyl)ethyl]resorcinol, 4-[2-(4-methylphenyl)ethyl]resorcinol, 4-[1-(4-methylphenyl)propyl]resorcinol, 4-[1-(4-methylphenyl)butyl]resorcinol, 4-[1-(4-methylphenyl)hexyl]resorcinol, 4-[1-(2-methoxyphenyl)ethyl]resorcinol, 4-[1-(3-methoxyphenyl)ethyl]resorcinol, 4-[1-(4-methoxyphenyl)ethyl]resorcinol, 4-[2-(2-methoxyphenyl)ethyl]resorcinol, 4-[2-(3-methoxyphenyl)ethyl]resorcinol, 4-[2-(4-methoxyphenyl)ethyl]resorcinol, 4-[1-(4-methoxyphenyl)propyl]resorcinol, 4-[1-(4-methoxyphenyl)butyl]resorcinol, 4-[1-(4-methoxyphenyl)hexyl]resorcinol, 4-[1-(3,4-dimethylphenyl)ethyl]resorcinol, 4-[1-(3,4-dimethoxyphenyl)ethyl]resorcinol, 4-[2-(3,4-dimethylphenyl)ethyl]resorcinol, 4-[2-(3,4-dimethoxyphenyl)ethyl]resorcinol, 4-[1-(3-methoxy-4-methylphenyl)ethyl]resorcinol, 4-[2-(3-methoxy-4-methylphenyl)ethyl]resorcinol, 4-[1-(3-methyl-4-methoxyphenyl)ethyl]resorcinol, 4-[2-(3-methyl-4-methoxyphenyl)ethyl]resorcinol, 4-[1-(3-methoxy-4-methylphenyl)propyl]resorcinol, 4-[1-(3-methyl-4-methoxyphenyl)propyl]resorcinol, 4-[1-(3-methoxy-4-methylphenyl)butyl]resorcinol, 4-[1-(3-methyl-4-methoxyphenyl)butyl]resorcinol, 4-[1-(3-methoxy-4-methylphenyl)hexyl]resorcinol, 4-[1-(3-methyl-4-methoxyphenyl)hexyl]resorcinol, 4-[1-(3,5-dimethoxy-4-methylphenyl)ethyl]resorcinol, 4-[2-(3,5-dimethoxy-4-methylphenyl)ethyl]resorcinol, 4-[1-(3,5-dimethoxy-4-methylphenyl)propyl]resorcinol, 4-[1-(3,5-dimethoxy-4-methylphenyl)butyl]resorcinol, 4-[1-(3,5-dimethoxy-4-methylphenyl)hexyl]resorcinol, 4-[1-(3,4,5-trimethoxyphenyl)ethyl]resorcinol, 4-[2-(3,4,5-trimethoxyphenyl)ethyl]resorcinol, 4-[1-(3,4,5-trimethoxyphenyl)propyl]resorcinol, 4-[1-(3,4,5-trimethoxyphenyl)butyl]resorcinol, 4-[1-(3,4,5-trimethoxyphenyl)hexyl]resorcinol and their mixtures.

The preferred resorcinols are 4-hexylresorcinol, 4-ethylresorcinol, 4-isopropylresorcinol, 4-butylresorcinol, and 4-(1-phenylethyl)resorcinol.
thereof.

More particular preferred resorcinol derivatives include 4-ethyl resorcinol, 4-isopropyl resorcinol, 4-hexyl resorcinol and 4-cyclohexyl resorcinol, as well as O-acylated versions thereof. Most preferred resorcinol derivatives are 4-ethyl resorcinol and 4-hexyl resorcinol.

### 4-Cycloalkyl Resorcinols

In the case resorcinol derivatives of formula (I) where R₃ represents a structure of the general formula (II) shown hereinabove, the resorcinol derivatives are referred to herein as 4-cycloalkyl resorcinols, and are represented by the general formula (III):

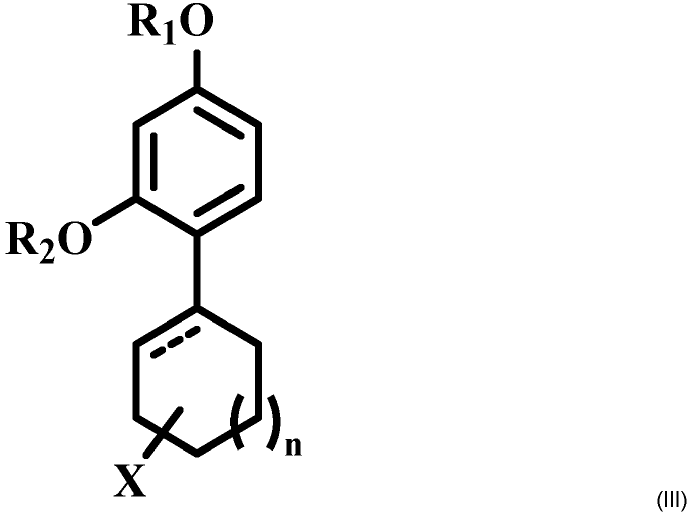

In the general formula (III) (as well as formula (II) hereinabove):
X represents hydrogen; OR¹, wherein R¹ represents hydrogen, (C₁ - C₆)alkyl or aryl-(C₁-C₆)alkyl; OCOR² wherein R₂ represents (C₁-C₆)alkyl, aryl-(C₁-C₆)alkyl or phenyl; (C₁-C₆)alkyl;
aryl-(C₁-C₆)alkyl; or NHR¹ wherein R¹ is defined as above;
n is 0 to 3; and the dashed line indicates an optional double bond at that position.

Examples of more specific embodiments of the 4-cycloalkyl resorcinols include:
(a) compounds of the formula (III) wherein a single bond connects the two carbon atoms at the dashed line;
(b) compounds of the formula (III) wherein n is one;
(c) compounds of the formula (III) wherein X is hydrogen;
(d) compounds of the formula (III) wherein X is hydrogen, methyl or ethyl;
(e) compounds of the formula (III) wherein n is zero;
(f) compounds of the formula (III) wherein n is two; and
(g) compounds of the formula (III) wherein X is benzyloxy.

Preferred compounds of formula (III) are 4-cyclohexyl resorcinol, 4-cycloheptyl resorcinol, and 4-cyclooctyl resorcinol. Most preferred compound of formula (III) is 4-cyclohexyl resorcinol.

The amount of the resorcinol derivative is in the range of about 0.00001 wt % to about 10 wt%, preferably about 0.001 to about 7 wt %, most preferably about 0.01 to about 5 wt%, by weight of the total amount of a cosmetic composition.

### NDAC

We have now found that NDAC inhibits the oxidation of 4-substituted resorcinol derivatives. We have now also demonstrated the preventive effect of NDAC on resorcinol derivatives induced color formation in cosmetic vehicle or cosmetic composition. Preferably, in accordance with the present invention, NDAC is used to stabilize 4-substituted resorcinols incorporated in cosmetic compositions.

Moreover, NDAC advantageously achieves the stabilization with no sulfurous odor generation as compared with other sulfurous odor generating cystine derivatives. Examples of the other sulfurous odor generating cystine derivatives which are less advantageous than NDAC are N-Acetyl Cysteine ("NACys") which contains a thiol (SH) group and Cystine diethyl ester ("DEC") which does not contain an SH-group.

NDAC is an amide, i.e., an N,N'-diacetyl derivative of cystine. NDAC, for purposes of the present invention, has the following chemical structure: 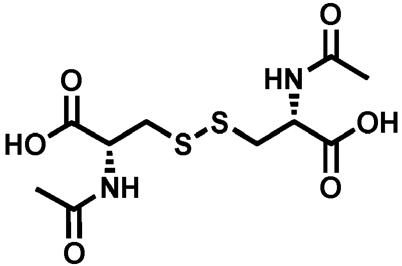 The molecular weight of NDAC = 324.4.

NDAC stereoisomers (referring to the stereoisomerism of the alpha-Carbon atom located between the Nitrogen atom and the Carbonyl group of the carboxylic acid moiety) of the present invention include R,R (L-cystine), R,S, S,R and S,S (D-cystine). Preferably, L stereoisomers are employed, and this is the most abundant and natural isomeric form found in nature.

NDAC is not readily commercially available but may be sourced on lab scale. NDAC may be synthesized directly from NACys as described in Vandana Rathore et al, Organic Letters, 20(19), 6274-6278; 2018 and Scott J. Pye et al, Green Chemistry, 20(1), 118-124; 2018:

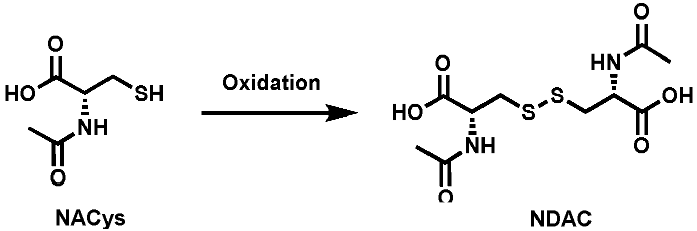

NDAC is obtained when NACys is dimerized via a S-S disulfide bridge. NACys is a thiol drug used commonly as an expectorant (CrystEngComm, 2016, 18, 8587, referred to as "NACe" therein). As will be seen from the Examples presented below, NACys is also effective at stabilizing resorcinol derivatives, however, it provides an upleasant sulfurous odor, that is unacceptable in cosmetic products. Furthermore, the sulfurous odor (monitored as hydrogen sulfide or H₂S) is consistent with NACys decomposition which is also unacceptable in marketed cosmetic products. The particular advantage of the inventive compositions is that 4-substituted resorcinols can be stabilized by NDAC against oxidation.

An amount of NDAC effective to inhibit the oxidation of 4-substituted resorcinol derivative may be determined by experimentation. NDAC and 4-substituted resorcinol derivatives are present in the composition in a weight ratio of 1:10000 to 10000:1 of NDAC :resorcinol, preferably 1:1000 to 1:5000, more preferably 1:1 to 1:1000.

Preferably, the amount of NDAC in the cosmetic composition is in the range of about 0.00001 % to about 10 wt %, more preferably about 0.0001 % to about 5 wt %, most preferably about 0.0001 % to about 2 wt %). As will be seen from the Examples presented below, NDAC is effective at inhibiting the discoloration of 4-substituted resorcinols and stabilizing compositions containing them.

### OPTIONAL COMPONENTS

Any of the compounds described herein can be mixed with other components. Examples of such components include oily components such as hydrocarbons, fats and oils such as liquid paraffin, squalene, petroleum jelly such as Vaseline^{®} (a registered trademark of Conopco Corp., Englewood Cliffs, New Jersey), cetyl alcohol, isostearyl alcohol, cetyl-2-ethylhexanoate, 2-octyldodecyl alcohol, glycerin, glycerin triisostearate, nut oils, and lanolin, as well as wax, silicone, surfactants, thickeners, neutralizers, antiseptics, germicides, anti-oxidants, powder components, pigments, perfumes, ultraviolet light absorbents, drugs, metallic sealant, and pH modifiers.

### SKIN BENEFIT AGENTS

The cosmetic compositions of the present invention may be used in personal care and personal wash products, such as, for example, lotions, soaps, deodorants, etc.

Preferred personal care compositions are those suitable for the application to human skin, which optionally, but preferably, include a skin benefit agent. Suitable skin benefit agents include anti-aging, wrinkle-reducing, bioenergetic boosting, skin whitening, anti-acne, and sebum reduction agents. Examples of these include alpha-hydroxy acids and esters, beta-hydroxy acids and esters, polyhydroxy acids and esters, kojic acid and esters, ferulic acid and ferulate derivatives, vanillic acid and esters, dioic acids (such as sebacic and azoleic acids) and esters, retinol, retinal, retinyl esters, hydroquinone, t-butyl hydroquinone, mulberry extract, licorice extract, niacinamide and its derivatives, N-acetylmethionine (AceMet) and 1-methylnicotinamide chloride, and resorcinol derivatives other than the 4-substituted resorcinol derivatives discussed hereinabove.

### Niacinamide and its Derivatives

The NDAC and Resorcinol active compounds described herein can be used in combination with Niacinamide. Niacinamide, also known as nicotinamide and as pyridine-3-carboxamide is the active, water soluble form of vitamin B3. It is essential to the coenzymes NADH and NADPH and therefore for over 200 enzymatic reactions in the body including ATP formation.

Niacinamide is present in the composition of the present invention in a concentration of 0.1 to 10 wt%, preferably 0.5 to 9 wt%, more preferably 1 to 8 wt%, still more preferably 2 to 7 wt%, even more preferably 3 to 8 wt%, yet more preferably 4 to 7 wt%, or even 5 to 6 wt% by weight of the composition.

Niacinamide is available for example from Sigma-Aldrich (Cat No:N0636), as are its derivative 1-methylnicotinamide chloride (Cat No: SML0704).

The active compounds described herein can also be used in combination with skin peeling agents (including glycolic acid or trichloroacetic acid face peels) or skin exfoliating agents (including retinoids, such as retinoic acid or retinol) to lighten skin tone and prevent repigmentation. Preferably, the active compound is administered in an amount and at an interval that results in the desired treatment of or improvement in the disorder or condition being treated.

An active compound described herein can also be used in combination with sunscreens (UVA or UVB blockers, absorbers, diffusing agents) to prevent or protect against sun or UV-induced skin damage.

In some variations, the composition further comprises ascorbic acid, its derivatives and ascorbic-acid based products (such as magnesium ascorbate) or other products with an anti-oxidant mechanism (such as resveratrol, tocopherols, tocotrienols and derivatives).

In some variations, the composition further comprises a soybean extract that is a blend of compounds isolated from soybean. The soybean extract may contain only a portion of the soybean (e.g., an extract of the soybean such as a lipid reduced soybean powder or filtered soymilk) or may contain the entire soybean (e.g., a ground powder of the soybean). The soybean extract may be in the form of a fluid (e.g., soymilk) or a solid (e.g., a soybean powder or soymilk powder).

In the cosmetic compositions provided herein, the concentration of the active compound is generally between 0.01% and 10%, or between about 0.01% and about 10%, for example between 0.1% and 5% or between about 0.1% and about 5%, or between 0.1% and 2%, or between about 0.1% and about 2%, or between 0.1% and 1%, or between about 0.1% and about 1%, relative to the total weight of the composition.

The compositions described herein can be applied directly to the skin. Alternatively, they can be delivered by various transdermal delivery systems, such as transdermal patches as known in the art. For example, for topical administration, the active ingredient can be formulated in a solution, gel, lotion, ointment, cream, suspension, paste, liniment, powder, tincture, aerosol, patch, or the like in a cosmetically acceptable form by methods known in the art. The composition can be any of a variety of forms common in the cosmetic arts for topical application to animals or humans, including solutions, lotions, sprays, creams, ointments, salves, gels, etc., as described below. Exemplary agents are those that are viscous enough to remain on the treated area, those that do not readily evaporate, and/or those that are easily removed by rinsing with water, optionally with the aid of soaps, cleansers and/or shampoos.

The compositions may be made into a wide variety of product types that include but are not limited to solutions, suspensions, lotions, creams, gels, toners, sticks, sprays, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks, make-up such as foundations, eye liners, and eye shadows, and the like. These product types may contain several types of cosmetically-acceptable carriers including, but not limited to solutions, suspensions, emulsions such as microemulsions and nano-emulsions, gels, solids and liposomes.

### COSMETICALLY ACCEPTABLE VEHICLE

The cosmetically acceptable vehicle may act as a dilutant, dispersant or carrier for the skin benefit ingredients in the composition, so as to facilitate their distribution when the composition is applied to the skin.

The vehicle may be aqueous, anhydrous or an emulsion. Preferably, the compositions are aqueous or an emulsion, especially water-in-oil or oil-in-water emulsion, preferentially oil in water emulsion. Water when present will be in amounts which may range from 5 to 99%, preferably from 20 to 70%, optimally between 40 and 70% by weight.

Besides water, relatively volatile solvents may also serve as carriers within compositions of the present invention. Most preferred are monohydric C₁-C₃ alkanols. These include ethyl alcohol, methyl alcohol and isopropyl alcohol. The amount of monohydric alkanol may range from 1 to 70%, preferably from 10 to 50%, optimally between 15 to 40% by weight.

Emollient materials may also serve as cosmetically acceptable carriers. These may be in the form of silicone oils and synthetic esters. Amounts of the emollients may range anywhere from 0.1 to 50%, preferably between 1 and 20% by weight.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, preferably from 4 to 5, silicon atoms. Linear volatile silicone materials generally have viscosities less than about 5 centistokes at 25°C while cyclic materials typically have viscosities of less than about 10 centistokes. Nonvolatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 to about 25 million centistokes at 25°C. Among the preferred non-volatile emollients useful in the present compositions are the polydimethyl siloxanes having viscosities from about 10 to about 400 centistokes at 25°C.

Among the ester emollients are:
(1)Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
(2)Ether-esters such as fatty acid esters of ethoxylated fatty alcohols.
(3)Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4)Wax esters such as beeswax, spermaceti, myristyl myristate, stearyl stearate and arachidyl behenate.
(5)Sterols esters, of which cholesterol fatty acid esters are examples.

Fatty acids having from 10 to 30 carbon atoms may also be included as cosmetically acceptable carriers for compositions of this invention. Illustrative of this category are pelargonic, lauric, myristic, palmitic, stearic, isostearic, hydroxystearic, oleic, linoleic, ricinoleic, arachidic, behenic and erucic acids.

Humectants of the polyhydric alcohol-type may also be employed as cosmetically acceptable carriers in compositions of this invention. The humectant aids in increasing the effectiveness of the emollient, reduces skin dryness and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. The amount of humectant may range anywhere from 0.5 to 30%, preferably between 1 and 15% by weight of the composition.

Thickeners may also be utilized as part of the cosmetically acceptable carrier of compositions according to the present invention. Typical thickeners include crosslinked acrylates (e.g. Carbopol 982), hydrophobically-modified acrylates (e.g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.0001 to 5%, usually from 0.001 to 1%, optimally from 0.01 to 0.5% by weight.

Collectively the water, solvents, silicones, esters, fatty acids, humectants and/or thickeners will constitute the cosmetically acceptable carrier in amounts from 1 to 99.9%, preferably from 80 to 99% by weight.

An oil or oily material may be present, together with an emulsifier to provide either a water-in-oil emulsion or an oil-in-water emulsion, depending largely on the average hydrophilic-lipophilic balance (HLB) of the emulsifier employed.

The compositions can be formulated as solutions. Solutions typically include an aqueous or organic solvent, e.g., from about 50% to about 99.99 wt% or from about 90% to about 99 wt% of a cosmetically acceptable aqueous or organic solvent. Examples of suitable organic solvents include: propylene glycol, polyethylene glycol (200-600), polypropylene glycol (425-2025), glycerol, 1,2,4-butanetriol, sorbitol esters, 1,2,6-hexanetriol, ethanol, and mixtures thereof. One example of such solvents is a mixture of ethanol/polyethylene glycol (80/20).

A lotion can be made from such a solution. Lotions typically contain from about 1% to about 20 wt% (e.g., from about 5% to about 10 wt%) of an emollient(s) and from about 50% to about 90 wt% (e.g., from about 60% to about 80 wt%) of water.

Another type of product that may be formulated from a solution is a cream. A cream typically contains from about 5% to about 50 wt% (e.g., from about 10% to about 20 wt%) of an emollient(s) and from about 45% to about 85 wt% (e.g., from about 50% to about 75 wt%) of water.

Yet another type of product that may be formulated from a solution is an ointment. An ointment may contain a simple base of animal, vegetable, or synthetic oils or semi-solid hydrocarbons. An ointment may contain from about 2% to about 10 wt% of an emollient(s) plus from about 0.1% to about 2 wt% of a thickening agent(s). Examples of thickening agents include, but are not limited to, those set forth in the ICI Handbook (Intemational Cosmetic Ingredient Dictionary and Handbook) pp. 1693-1697.

The compositions described herein can also be formulated as emulsions. If the carrier is an emulsion, from about 1% to about 10 wt% (e.g., from about 2% to about 5 wt%) of the carrier contains an emulsifier(s). Emulsifiers may be nonionic, anionic or cationic. Examples of emulsifiers include, but are not limited to, those set forth in the ICI Handbook, pp. 1673-1686.

Lotions and creams can be formulated as emulsions. Typically such lotions contain from 0.5% to about 5 wt% of an emulsifier(s), while such creams would typically contain from about 1% to about 20 wt% (e.g., from about 5% to about 10 wt%) of an emollient(s); from about 20% to about 80 wt% (e.g., from 30% to about 70 wt%) of water; and from about 1% to about 10 wt% (e.g., from about 2% to about 5 wt%) of an emulsifier(s).

Single emulsion skin care preparations, such as lotions and creams, of the oil-in-water type and water-in-oil type are well-known in the art and are useful in compositions and methods described herein. Multiphase emulsion compositions, such as the water-in-oil-in-water type or the oil-in-water-in-oil type, are also useful in the compositions and methods describe herein. In general, such single or multiphase emulsions contain water, emollients, and emulsifiers as essential ingredients.

The compositions described herein can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

One or more additional agents can be added in the topical formulations in order to enhance the percutaneous absorption of the active ingredients, including, but not limited to, dimethylsulfoxide, dimethylacetamide, dimethylformamide, surfactants, azone (laurocapram), alcohol, acetone, propylene glycol and polyethylene glycol. Physical methods can also be used to enhance transdermal penetration such as iontophoresis or sonophoresis.

A topically applied composition provided herein contains a cosmetically effective agent that has the desired effect on skin as described herein, and those ingredients as are necessary for use as a carrier, such as an emulsion, a cream, an ointment, an aqueous solution, a lotion or an aerosol.

The carrier utilized in the compositions described herein can be in a wide variety of forms. These include emulsion carriers, including, but not limited to, oil-in-water, water-in-oil, water-in-oil-in-water, and oil-in-water-in-silicone emulsions, a cream, an ointment, an aqueous solution, a lotion or an aerosol. A given component will distribute primarily into either the water or oil/silicone phase, depending on the water solubility/dispersibility of the component in the composition.

An emollient may also be added to the cosmetic/dermatological compositions described herein. The emollient component can comprise fats, oils, fatty alcohols, fatty acids and esters which aid application and adhesion, yield gloss and provide occlusive moisturization. Suitable emollients for use may be isostearic acid derivatives, isopropyl palmitate, lanolin oil, diisopropyl dimerate, maleated soybean oil, octyl palmitate, isopropyl isostearate, cetyl lactate, cetyl ricinoleate, tocopheryl acetate, acetylated lanolin alcohol, cetyl acetate, phenyl trimethicone, glyceryl oleate, tocopheryl linoleate, wheat germ glycerides, arachidyl propionate, myristyl lactate, decyl oleate, propylene glycol ricinoleate, isopropyl linoleate, pentaerythrityl tetrastearate, neopentylglycol dicaprylate/dicaprate, hydrogenated coco-glycerides, isononyl isononanoate, isotridecyl isononanoate, myristyl myristate, triisocetyl citrate, cetyl alcohol, octyl dodecanol, oleyl alcohol, panthenol, lanolin alcohol, linoleic acid, linolenic acid, sucrose esters of fatty acids, octyl hydroxystearate and mixtures thereof. Suitable emollients may include polar emollient emulsifiers (such as linear or branched chained polyglycerol esters) and non-polar emollients. The emollient component typically may comprise from about 1% to about 90%, preferably from about 10% to about 80%, more preferably from about 20% to about 70%, and most preferably from about 40% to about 60%, by weight of the cosmetic composition.

By "polar emollient," as used herein, is meant any emollient emulsifier having at least one polar moiety and wherein the solubility (at 30 °C) of the cytoprotective derivative compound in the polar emollient is greater than about 1.5%, preferably greater than about 2%, more preferably greater than about 3% by weight. Suitable polar emollients may include, but are not limited to, polyol ester and polyol ethers such as linear or branched chained polyglycerol esters and polyglycerol ethers. Non-limiting examples of such emollients may include PG3 diisostearate, polyglyceryl-2-sesquiisostearate, polyglyceryl-5-distearate, polyglyceryl-10-distearate, polyglyceryl-10-diisostearate, acetylated monoglycerides, glycerol esters, glycerol tricaprylate/caprate, glyceryl ricinoleate, glyceryl isostearate, glyceryl myristate, glyceryl linoleate, polyalkylene glycols such as PEG 600, monoglycerides, 2-monolaurin, sorbitan esters and mixtures thereof.

By "non-polar emollient," as used herein, means any emollient emulsifier possessing no or minimal permanent electric moments. Suitable non-polar emollients may include, but are not limited to, esters and linear or branched chained hydrocarbons. Non-limiting examples of such emollients may include isononyl isononanoate, isopropyl isostearate, octyl hydroxystearate, diisopropyl dimerate, lanolin oil, octyl palmitate, isopropyl palmitate, paraffins, isoparaffins, acetylated lanolin, sucrose fatty acid esters, isopropyl myristate, isopropyl stearate, mineral oil, silicone oils, dimethicone, allantoin, isohexadecane, isododecane, petrolatum, and mixtures thereof. The solubility of the compound in polar or non-polar emollients may be determined according to methods known in the art.

Suitable oils include esters, triglycerides, hydrocarbons and silicones. These can be a single material or a mixture of one or more materials. They may normally comprise from 0.1% to about 100%, preferably from about 5% to about 90%, and most preferably from about 70% to about 90% by weight of the emollient component.

Further, suitable oils for use herein may be acetylglycerides, octanoates, and decanoates of alcohols and polyalcohols, such as those of glycol and glycerol, the ricinoleates of alcohols and polyalcohols such as cetyl ricinoleate, PG-3 diisostearate, polyglycerol ethers, polyglyerol esters, caprylic triglycerides, capric triglycerides, isostearic triglyceride, adipic triglyceride, phenyl trimethicone, lanolin oil, polybutene, isopropyl palmitate, isopropyl isostearate, cetyl ricinoleate, octyl dodecanol, oleyl alcohol, hydrogenated vegetable oils, castor oil, modified lanolins, octyl palmitate, lanolin oil, maleated soybean oil, cetyl ricinoleate, glyceryl trioctanoate, diisopropyl dimerate, synthetic lanolin derivatives and branched chain alcohols, sucrose esters of fatty acids, octyl hydroxystearate and mixtures thereof.

A surfactant may also be added to compositions described herein, in order to confer beneficial cosmetic or application properties. Surfactants suitable for use may be those which can form emulsions and/or association structures.

For leave-on products, total concentration of the surfactant will range from 0.1 to 40%, preferably from 1 to 20%, optimally from 1 to 5% by weight of the composition. For wash-off products, such as cleansers and soap, total concentration of surfactant will range at about 1 to about 90 wt%.

The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Examples of surface active agents which may be used in the compositions described herein include sodium alkyl sulfates, e.g., sodium lauryl sulfate and sodium myristyl sulfate, sodium N-acyl sarcosinates, e.g., sodium N-lauroyl sarcosinate and sodium N-myristoyl sarcosinate, sodium dodecylbenzenesulfonate, sodium hydrogenated coconut fatty acid monoglyceride sulfate, sodium lauryl sulfoacetate and N-acyl glutamates, e.g., N-palmitoyl glutamate, N-methylacyltaurin sodium salt, N-methylacylalanine sodium salt, sodium alpha-olefin sulfonate and sodium dioctylsulfosuccinate; N-alkylaminoglycerols, e.g., N-lauryl-diamino-ethylglycerol and N-myristyldiaminoethylglycerol, N-alkyl-N-carboxymethylammonium betaine and sodium 2-alkyl-1-hydroxyethylimidazoline betaine; polyoxyethylenealkyl ether, polyoxyethylenealkylaryl ether, polyoxyethylenelanolin alcohol, polyoxyethyleneglyceryl monoaliphatic acid ester, polyoxyethylenesorbitol aliphatic acid ester, polyoxyethylene aliphatic acid ester, higher aliphatic acid glycerol ester, sorbitan aliphatic acid ester, Pluronic type surface active agent, and polyoxyethylenesorbitan aliphatic acid esters such as polyoxyethylenesorbitan monooleate and polyoxyethylenesorbitan monolaurate. Emulsifier-type surfactants known to those of skill in the art can be used in the compositions described herein.

Also useful herein may be surfactants that form association structures, preferably lamellar or hexagonal liquid crystals, at ambient temperature when mixed with a polar solvent. In preparing a sample combination of surfactant and polar solvent to demonstrate the ability to form association structures, the surfactant needs to be sufficiently soluble in the polar solvent such that an association structure can form at ambient temperature.

Any surfactant which forms association structures at ambient temperature and is suitable for use in cosmetics may be suitable for use herein. Surfactants suitable for use in cosmetics present no or minimal dermatological or toxicological problems. Anionic surfactants, nonionic surfactants, cationic surfactants, amphoteric surfactants and mixtures thereof may be suitable for use.

The surfactants can be used at levels from about 4% to about 97%, preferably from about 5% to about 95%, more preferably from about 20% to about 90% and most preferably from about 30% to about 70% by weight of the association structure.

The cosmetic compositions described herein may contain one or more materials, herein singly or collectively referred to as a "solidifying agent", that are effective to solidify the particular liquid base materials to be used in a cosmetic composition. (As used herein, the term "solidify" refers to the physical and/or chemical alteration of the liquid base material so as to form a solid or semi-solid at ambient conditions, i.e., to form a final composition that has a stable physical structure and can be deposited on the skin under normal use conditions.) As is appreciated by those skilled in the art, the selection of the particular solidifying agent for use in the cosmetic compositions will depend upon the particular type of composition desired, i.e., gel or wax-based, the desired rheology, the liquid base material used and the other materials to be used in the composition. The solidifying agent can be preferably present at a concentration of from about 0.1% to about 90%, more preferably from about 1% to about 50%, even more preferably from about 5% to about 40%, most preferably from about 3% to about 20% by weight.

The wax cosmetic stick variations provided herein preferably may contain from about 5% to about 50% (by weight) of a waxy solidifying agent. By the term "waxy solidifying agent," as used herein, is meant a solidifying material having wax-like characteristics. Such waxy materials may also serve as emollients. Among the waxy materials useful herein are the high melting point waxes, i.e., having a melting point of from about 65°C to about 125°C, such as beeswax, spermaceti, carnauba, bayberry, candelilla, montan, ozokerite, ceresin, paraffin, synthetic waxes such as Fisher-Tropsch waxes, microcrystalline wax, and mixtures thereof. Ceresin, ozokerite, white beeswax, synthetic waxes, and mixtures thereof, are among those useful herein. Low melting waxes, having a melting point of from about 37° C to about 75° C, may be preferred for use in the wax stick variations described herein. Wax stick variations, which contain volatile silicone oils as a liquid base material, preferably contain from about 10% to about 35%, more preferably from about 10% to about 20% (by weight), of a low-melting wax. Such materials include fatty acids, fatty alcohols, fatty acid esters and fatty acid amides, having fatty chains of from about 8 to about 30 carbon atoms, and mixtures thereof. Wax-like materials include cetyl alcohol, palmitic acid, stearyl alcohol, behenamide, sucrose esters of tallow fatty acids, mono and di-fatty acid esters of polyethylene glycol, and mixtures thereof. Stearyl alcohol, cetyl alcohol, and mixtures thereof, are mostly used. Additional fatty acids, fatty alcohols, and other wax-like materials are also well known in the art.

In addition, these compositions may include other cosmetic agents, carriers, adjuvants, and the like. Some particular additional agents may include sunscreens; retinoids; antioxidants; hydroxyacids; fatty acids, acceptable non-toxic metal salts of naturally occurring amino acids or of hydroxyalkyl acids; botanical extracts, salicylic acid, benzoyl peroxide, antibiotics, antiandrogens, anti-inflammatory agents, antioxidants, ascorbic acid, vitamins B, tocopherols or tocotrienols, corticosteroids, moisteners, surfactants, keratolytic agents, complexing agents, colorants, fragrances, and mixtures thereof.

### EXAMPLES

### EXAMPLE I

### Evaluation for Signs of Degradation and Stabilization of 4-Alkyl Resorcinols

Test reagents: 4-ethylresorcinol and 4-hexylresorcinol were purchased from Sigma.

Niacinamide (B3) was purchased from Sigma.

N-Acetyl-L-cysteine (NACys) was purchased from Sigma. N,N-Diacetyl-L-cystine (NDAC) was purchased from CombiBlocks.

### Color and Odor Measurements

Samples were prepared by dissolving 4-hexylresorcinol (HR) (200mg) or 4-ethylresorcinol (ER) (200mg) in DI water:butylene glycol (4:1; 20ml) to generate a clear colorless homogeneous solution. A portion of each solution (5ml) was added to a 20ml scintilation vial containing either niacinamide (B3) (50mg), NDAC (N,N'-diacetyl-L-cystine) (50mg), NACys (N-acetyl-L-cysteine) (50mg), B3 (50mg) + NDAC (50mg) or B3 (50mg) + NACys (50mg). A hydrogen sulfide (H₂S) test strip (Sigma) was placed above the sample solution and the samples sealed. Odor and H₂S generation (unchanged test strip white color = no H₂S generated; changed test strip brown/black color = H₂S generated) were monitored for 2 weeks at R.T. (about 20 to about 22 °C).

After 2 weeks of storage at R.T. (about 20 to about 22 °C), the color of each solution was assessed visually. Additionally, L*a*b* measurements determined experimentally using a Labscan XE instrument (Hunter Associates Labs Inc., Reston, VA) and processed with Universal Software (version 4.10). The L* parameter measures darkness and lightness and ranges from black (L* = 0) to white (L* = 100). The a* parameter measures color content and intensity ranging from green (a* < 0) to neutral grey (a* = 0) to red (a* > 0). The b* parameter also measures color content and intensity ranging from blue (b* < 0) to neutral grey (b* = 0) to yellow (b* > 0).

### Quantitation of 4-Hexylresorcinol and (HR) 4-ethylresorcinol (ER)

The amount of 4-hexylresorcinol (HR) or 4-ethylresorcinol (ER) present in each of the samples used for the color measurements was quantitated after 2 weeks @ R.T. (~20-22 °C) by high performance liquid chromatography (HPLC). A sample aliquot (100uL) from each of the color measurement solutions was diluted with DI water: acetonitrile (1:1, 900uL) to generate test samples (1ml). An aliquot (10uL) from each test sample was injected into a Waters 2695 Separations Module, chromatographed and separated on a Restek Pinnacle DB C18 (5um, 4.6 X 150mm) column operated at 30 °C and the 4-alkylresorcinols monitored using a Waters 2996 Photodiode Array Detector set @ 281nm. The mobile phase consisted of water (A) and acetonitrile (B) and optimum separation was achieved using a gradient from 95:5 (A:B) to 5:95 (A:B) in 20min, followed by isocratic elution at 5:95 (A:B) for 3min at a flow rate of 1ml/min. The chromatographic data was processed using Empower 2 software (Waters Corporation, Milford, MA). Standard control samples of HR at 1X, 5X higher and 5X lower concentrations were prepared fresh and immediately analysed; standard curves were generated within the test sample range and linearity was confirmed.

All samples were analyzed in duplicate and the mean value reported. The amount of 4-alkylresorcinol present for each test sample after 2 weeks was determined by measuring the chromatographic peak area under the curve @ 281nm for HR and expressed as the percent ratio over the corresponding chromatographic peak area under the curve @ 281nm for the freshly prepared control sample.

Effect of NDAC and NACys on Color, Odor and Stability of 4-alkyl resorcinols in the presence and absence of B3 after 2 weeks at R.T. (~20-22 °C):

**TABLE 1**

| **Sample Number** | Sample ^{a} | Color Appearance ^{b} | **L*** | **a*** | **b*** | Odor | **Resorcinol Remaining** (% of control) ^{c} |
|---|---|---|---|---|---|---|---|
| 1 | Control | colorless | 100 | 0 | 0 | Non-sulforous | 100 |
| 2 | 1% HR | reddish pink | 80 | 20 | 20 | Non-sulforous | 61 |
| 3 | 1% HR + 1% B3 | orange | 80 | 15 | 35 | Non-sulforous | 44 |
| 4 | 1%HR+1% NDAC | nearly colorless | 95 | -2 | 15 | Non-sulforous | 101 |
| 5 | 1% HR + 1% B3 + 1% NDAC | nearly colorless | 95 | 0 | 15 | Non-sulforous | 99 |
| 6 | 1% HR + 0.2% NDAC | nearly colorless | 98 | -5 | 12 | Non-sulforous | ND |
| 7 | 1% HR + 1% B3 + 0.2% NDAC | nearly colorless | 98 | 10 | 10 | Non-sulforous | ND |
| 8 | 0.4% HR | reddish pink | 80 | 20 | 20 | Non-sulforous | ND |
| 9 | 0.4% HR + 1% B3 | orange | 75 | 15 | 30 | Non-sulforous | ND |
| 10 | 0.4% HR + 0.2% NDAC | nearly colorless | 98 | -1 | 1 | Non-sulforous | ND |
| 11 | 0.4% HR + 1% B3 + 0.2% NDAC | nearly colorless | 98 | 2 | 5 | Non-sulforous | ND |
| 12 | 0.4% HR + 0.1% NDAC | nearly colorless | 98 | -1 | 1 | Non-sulforous | ND |
| 13 | 0.4% HR + 1% B3 + 0.1% NDAC | nearly colorless | 98 | 2 | 1 | Non-sulforous | ND |
| 14 | 0.4% HR + 0.01% NDAC | nearly colorless | 95 | 5 | 15 | Non-sulforous | ND |
| 15 | 0.4% HR + 1% B3 + 0.01% NDAC | nearly colorless | 95 | 10 | 20 | Non-sulforous | ND |
| 16 | 1%HR+1% NACys^{d} | nearly colorless | 95 | -2 | 10 | Sulforous | 102 |
| 17 | 1% HR + 1% B3 + 1% NACys^{d} | nearly colorless | 95 | -2 | 15 | Sulforous | 91 |
| 18 | 0.4% ER | yellow | 90 | 5 | 70 | Non-sulforous | ND |
| 19 | 0.4% ER + 1% B3 | yellow | 85 | 5 | 65 | Non-sulforous | ND |
| 20 | 0.4% ER + 0.1% NDAC | nearly colorless | 98 | -1 | 1 | Non-sulforous | ND |
| 21 | 0.4% ER + 1% B3 + 0.1% NDAC | nearly colorless | 98 | 2 | 1 | Non-sulforous | ND^{e} |

| | | | | | | | |
|---|---|---|---|---|---|---|---|
| ^{a} Control = DI water:butylene glycol (4:1). ^{b} Visual appearance of solution after 2 weeks. ^{c} Percent resorcinol remaining, determined by HPLC analysis. ^{d} Cysteine derivative NACys is N-acetyl-L-Cysteine ^{e} ND = not determined. | | | | | | | |

The Table above shows the effect of NDAC and NACys on the color, odor and stability of HR and ER (NDAC only) in the presence and absence of B3.

A freshly prepared solution of HR is colorless and no decomposition of HR is observed upon HPLC analysis. After 2 weeks, the color of the HR solution changes to a reddish-pink color as demonstrated by the increases in a* and b* components (as well as a decrease in L*) and the level of HR decreases by 39% (See Sample 2).

Addition of B3 to the HR solution significantly accelerates decomposition of HR by 56% and generates a more intense color (orange) as seen by further significant increases in a*, b* and decreases in L* (See Sample 3).

In contrast and unexpectedly, addition of NDAC to the HR solution (in a 1:1 weight ratio) significantly prevents color formation and HR decomposition in the presence or absence of B3 (See Samples 4-7).

Weight ratios anywhere from 0.025:1 to 1:1 of NDAC:HR using HR at 0.4% and 1% (by weight) significantly prevent color formation (See Samples 10-15).

Further, NDAC was also effective at preventing color formation induced by other 4-alkylresorcinols such as ER, in the presence or absence of B3 (See Samples 20-21 versus 18-19).

Lastly, the cysteine derivative NACys also prevents color formation and HR decomposition in the absence of B3 (See Sample 16) and less so in the presence of B3 (See Sample 17), however, it generates sulforous odor which is undesirable for personal care compositions.

These results clearly demonstrate the significant benefits of NDAC on odor, color, and chemical stabilization of 4-alkylresorcinols in the presence or absence of other cosmetic ingredients (such as B3) that can further accelerate such color formation and decomposition.

### EXAMPLE II. 4-Ethyl Resorcinol (ER) and NDAC

A topical cosmetic composition within the scope of the invention was prepared. A base formulation shown in the Table below was made by heating phase A ingredients to 70 to 85° C with stirring. Phase B ingredients were heated in a separate container to 70 to 85° C with stirring. Then, phase A was added into phase B while both phases were kept at 70 to 85° C. The mixture was stirred for at least 15 minutes at 70 to 85° C, then cooled. Phase C ingredients were added at 50° C, followed by Phase D ingredients added at 40 ° C.

**TABLE 2. Base Formulation**

| | a | b | |
|---|---|---|---|
| Ingredients | %wt. | %wt. | Phase |
| Isostearyl Palmitate | 6.00 | 6.00 | A |
| C12-C15 Alkyl Octanoate | 3.00 | 3.00 | A |
| PEG-100 Stearate | 2.00 | 2.00 | A |
| Glyceryl Hydroxystearate | 1.50 | 1.50 | A |
| Stearyl Alcohol | 1.50 | 1.50 | A |
| Stearic acid | 3.00 | 4.00 | A |
| Propylparaben | 0.10 | 0.10 | A |
| Dimethicone | 1.00 | 1.00 | A |
| Sorbitan Monostearate | 1.00 | 1.00 | A |
| Vitamin E acetate | 0.10 | 0.10 | A |
| Cholesterol | 0.50 | 0.50 | A |
| TEA, 99% | 1.20 | 1.20 | B |
| Xanthan gum | 0.20 | 0.20 | B |
| Hydroxyethylcellulose | 0.50 | 0.50 | B |
| Magnesium Aluminum Silicate | 0.60 | 0.60 | B |
| Disodium EDTA | 0.05 | 0.05 | B |
| Niacinamide | 0.05 | 0.05 | C |
| BHT | 0.10 | 0.10 | C |
| 4-ethyl resorcinol | 0.05 | 2.00 | C |
| NDAC | 0.0005 | 0.001 | D |
| Fragrance components | 0.50 | 0.50 | D |
| Water | BAL | BAL | B |
| Total | 100.00 | 100.00 | |

### EXAMPLE III

An additional cosmetic composition was prepared falling within the scope of the invention.

| **TABLE 3** | Wt% | Phase |
|---|---|---|
| water, DI | BAL | A |
| xanthan gum | 0.2 | A |
| magnesium aluminum silicate | 0.6 | A |
| methyl paraben | 0.15 | A |
| butylene glycol 1,3 | 3.0 | A |
| hydroxyethylcellulose | 0.5 | A |
| glycerine, USP | 2.0 | A |
| sodium stearoyl lactylate | 0.5 | B |
| triethanolamine | 1.2 | B |
| stearic acid | 3.0 | B |
| glyceryl hydroxystearate | 1.5 | B |
| stearyl alcohol | 1.5 | B |
| isostearyl palmitate | 6.0 | B |
| C12-15 alcohols octanoate | 3.0 | B |
| dimethicone | 1.0 | B |
| cholesterol NF | 0.5 | B |
| sorbitan stearate | 1.0 | B |
| Fragrance components | 1.0 | B |
| tocopheryl acetate | 0.1 | B |
| PEG-100 stearate | 2.0 | B |
| BHT | 0.10 | C |
| hydroxycaprylic acid | 0.1 | C |
| 4-cyclohexyl Resorcinol | 10.0 | C |
| alpha-bisabolol | 0.2 | C |
| Niacinamide | 0.05 | C |
| NDAC | 0.001 | D |

The composition in the Table above was prepared as follows:
1. Heat Phase A to 80°C while mixing.
2. Heat Phase B to 75°C in a separate container while mixing.
3. Add B to A and mix with heat maintained at 70 - 80°C for 15 min; then heat turned off and continued mixing for another 15 min.
4. At 50°C add Phase C and mix for 10 min.
5. At 40°C add Phase D and mix for 10 min.

### EXAMPLE IV

A set of additional compositions within the scope of the present invention were prepared by the method of Example II and are listed in the table below.

**TABLE 4. 4-Hexyl Resorcinol (HR) and NDAC**

| Ingredients | Phase | Examples (wt. %) | | | | | | | |
|---|---|---|---|---|---|---|---|---|---|
| | | 4 acid soap base | 5 | 6 | 7 | 8 | 9 | 10 | 11 |
| Stearic acid | A | 17.9 | 17.9 | 17.9 | 17.9 | 17.9 | 17.9 | 17.9 | 17.9 |
| Sodium cetearyl sulfate | A | | 2.2 | | 1 | 1.5 | 2 | 3 | 2 |
| Myrj 59 | A | | | 2 | 2 | 2 | 2 | 2 | 1 |
| Propylparaben | A | 0.1 | 0.1 | 0.1 | 0.1 | 0.1 | 0.1 | 0.1 | 0.1 |
| Disodium EDTA | A | 0.05 | 0.05 | 0.05 | 0.05 | 0.05 | 0.05 | 0.05 | 0.05 |
| Span 60 | A | | | 2 | 2 | 2 | 2 | 2 | 1 |
| BHT | C | 0.10 | 0.10 | 0.10 | 0.10 | 0.10 | 0.10 | 0.10 | 0.10 |
| 4-Hexyl resorcinol | C | 0.05 | 0.05 | 2.0 | 2.0 | 3.5 | 3.5 | 5.0 | 10.0 |
| NDAC | D | 0.001 | 0.001 | 0.01 | 0.01 | 0.5 | 0.5 | 0.001 | 0.01 |
| KOH, 22% | B | 2.20 | | | | | | | |
| Glycerin | B | 1 | 1 | 1 | 1 | 1 | 1 | 1 | 1 |
| Methylparaben | B | 0.15 | 0.15 | 0.15 | 0.15 | 0.15 | 0.15 | 0.15 | 0.15 |
| Water | B | BAL | BAL | BAL | BAL | BAL | BAL | BAL | BAL |

### EXAMPLE V

The formulations in the Table below were prepared in accordance with the method set forth in Example III hereinabove.

**TABLE 5: Formulations for Skin Lotions**

| | | | #1 | #2 | #3 | #4 | #5 |
|---|---|---|---|---|---|---|---|
| Raw Material | | | Wt % | Wt % | Wt % | Wt % | Wt % |
| | | | | | | | |
| Stearic acid | A | | 17.90 | 17.90 | 17.90 | 17.90 | 17.90 |
| etostearyl Alcohol | A | | 0.53 | 0.53 | 0.53 | 0.53 | 0.53 |
| Dimethicone (DC200, 350 cSt) | A | | 0.50 | 0.50 | 0.50 | 0.50 | 0.50 |
| Parsol MCX | A | | 1.25 | 1.25 | 1.25 | 1.25 | 1.25 |
| Parsol 1789 | A | | 0.40 | 0.40 | 0.40 | 0.40 | 0.40 |
| Propyl paraben | A | | 0.10 | 0.10 | 0.10 | 0.10 | 0.10 |
| | | | | | | | |
| Water | B | | 55.88 | 55.88 | 55.88 | 55.88 | 55.88 |
| Glycerin | B | | 1.00 | 1.00 | 1.00 | 1.00 | 1.00 |
| EDTA | B | | 0.04 | 0.04 | 0.04 | 0.04 | 0.04 |
| Methyl paraben | B | | 0.20 | 0.20 | 0.20 | 0.20 | 0.20 |
| KOH, 22% | B | | 2.20 | 2.20 | 2.20 | 2.20 | 2.20 |
| | | | | | | | |
| NDAC | D | | 1.0 | 1.0 | 1.0 | 1.0 | 1.0 |
| | | | | | | | |
| Niacinamide | C | | 1.00 | 1.00 | | -------- | 1.00 |
| 4-Ethyl Resorcinol | C | | 2.00 | 3.55 | | -------- | ------- |
| 4-Hexyl Resorcinol | C | | | -------- | 5.00 | ------- | ------- |
| 4-cyclohexyl Resorcinol | C | | | -------- | ------ | 4.00 | 1.00 |
| Q.S. to 100% with water | | | Q.S. | Q.S. | Q.S. | Q.S. | Q.S. |

It should be understood that the specific forms of the invention herein illustrated and described are intended to be representative only. Changes, including but not limited to those suggested in this specification, may be made in the illustrated embodiments without departing from the clear teachings of the disclosure.

## Claims

1. A topical cosmetic composition comprising:
a. 0.0001 to 10 wt % of N,N'-diacetyl-L-Cystine; preferably less than 1 to 2wt% of N,N'-diacetyl-L-Cystine; more preferably 0.2wt% of N,N'-diacetyl-L-Cystine;
b. 0.00001 to 10 wt% of one or more 4-substituted resorcinol derivatives of the general formula I; preferably 1-2wt% of a 4-substituted resorcinol derivative of the general formula I; more preferably 1wt% or less of a 4-substituted resorcinol derivative of the general formula I; alternatively 0.4wt% of a 4-substituted resorcinol derivative of the general formula I: 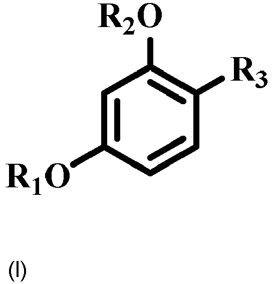
wherein each R₁ and R₂ independently is selected from the group consisting of a hydrogen atom, -CO-R, -COO-R_{,} and CONHR; wherein R represents a saturated or unsaturated, linear branched or cyclic C₁ - C₁₈ hydrocarbon;
wherein R₃ represents a saturated or unsaturated, linear, branched or cyclic C₁ - C₁₈ hydrocarbon;
and
c. a cosmetically acceptable vehicle.

2. The composition of claim 1, wherein both **R₁** and **R₂** are H.

3. The composition of any one of claims 1-2, wherein **R₃** is selected from saturated or unsaturated, linear, branched or cyclic C₂ - C₁₂ hydrocarbons.

4. The composition of any one of claims 1-3, wherein said 4-substituted resorcinol derivative of general formula I is selected from the group consisting of 4-ethyl resorcinol, 4-isopropyl resorcinol, 4-hexyl resorcinol, 4- cyclohexyl resorcinol, and mixtures thereof, or cosmetically acceptable salt thereof.

5. The composition of any one of claims 1-4, wherein **R₃** is hexyl or cyclohexyl.

6. The composition of any one of claims 1-5, wherein **R₃** is ethyl.

7. The cosmetic composition of any one of claims 1-6, further comprising a skin benefit agent selected from the group consisting of alpha-hydroxy acids and esters, beta-hydroxy acids and esters, polyhydroxy acids and esters, kojic acid and esters, ferulic acid and ferulate derivatives, vanillic acid and esters, dioic acids and esters, retinol, retinal, retinyl esters, hydroquinone, t-butyl hydroquinone, other resorcinol derivatives, niacinamide, N-acetylmethionine, 1-methylnicotinamide chloride, and mixtures thereof.

8. A cosmetic composition stabilized for storage comprising:
a. 0.1 to 2 wt % of a 4-substituted resorcinol derivative of the general formula I: 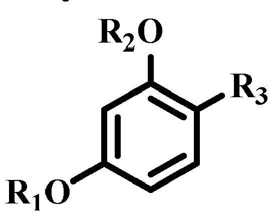 wherein each R₁ and R₂ independently is selected from the group consisting of a hydrogen atom, -CO-R, - COO-R, and CONHR; wherein R represents a saturated or unsaturated, linear branched or cyclic C₁ - C₁₈ hydrocarbon;
wherein R₃ represents a saturated or unsaturated, linear, branched or cyclic C₁ - C₁₈ hydrocarbon;
b. 0.0001 wt % to 5 wt % of N,N'-diacetyl-L-Cystine;
c. an optional skin benefit agent selected from the group consisting of alpha-hydroxy acids and esters, beta-hydroxy acids and esters, polyhydroxy acids and esters, kojic acid and esters, ferulic acid and ferulate derivatives, vanillic acid and esters, dioic acids and esters, retinol, retinal, retinyl esters, hydroquinone, t-butyl hydroquinone, other resorcinol derivatives, niacinamide, N-acetylmethionine, 1-methylnicotinamide chloride, and mixtures thereof; and
d. a cosmetically acceptable vehicle;
wherein the weight ratio of said N,N'-diacetyl-L-Cystine to said 4-substituted resorcinol derivative is 10,000:1 to 1:100,000; and
wherein said N,N'-diacetyl-L-Cystine stabilizes said 4-substituted resorcinol derivative, or said optional skin benefit agent, or both, in said vehicle.

9. The stabilized cosmetic composition according to claim 8, wherein said optional skin benefit agent is niacinamide.

10. A method of stabilizing 4-ethyl resorcinol or 4-hexyl resorcinol in a composition comprising a cosmetically acceptable vehicle by adding to the composition N,N'-diacetyl-L-Cystine in an amount sufficient to stabilize said 4-ethyl resorcinol or 4-hexyl resorcinol.

11. A method of using the stabilized composition according to claim 9 for topical cosmetic application to skin.

12. A method of using N,N'-diacetyl-L-Cystine and 4-substituted resorcinol derivatives in the manufacture of a topical cosmetic composition according to any of the claims 1-7 and 8-9.

## Patentansprüche

1. Topische kosmetische Zusammensetzung, umfassend:
a. 0,0001 bis 10 Gew.-% N,N'-Diacetyl-L-Cystin, vorzugsweise weniger als 1 bis 2 Gew.-% N,N'-Diacetyl-L-Cystin, besonders bevorzugt 0,2 Gew.-% N,N'-Diacetyl-L-Cystin;
b. 0,00001 bis 10 Gew.-% eines oder mehrerer 4-substituierter Resorcin-Derivate der allgemeinen Formel I, vorzugsweise 1 bis 2 Gew.-% eines 4-substituierten Resorcin-Derivats der allgemeinen Formel I, besonders bevorzugt 1 Gew.-% oder weniger eines 4-substituierten Resorcin-Derivats der allgemeinen Formel I, alternativ 0,4 Gew.-% eines 4-substituierten Resorcin-Derivats der allgemeinen Formel I: 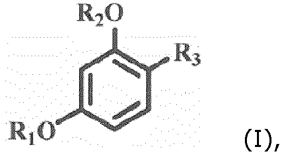
wobei R₁ und R₂ jeweils, unabhängig voneinander, aus der Gruppe ausgewählt sind, die aus einem Wasserstoffatom, -CO-R, -COO-R und CONHR besteht, wobei R einen gesättigten oder ungesättigten, linearen, verzweigten oder cyclischen C₁-C₁₈-Kohlenwasserstoff darstellt,
wobei R₃ einen gesättigten oder ungesättigten, linearen, verzweigten oder cyclischen C₁-C₁₈-Kohlenwasserstoff darstellt;
und
c. ein kosmetisch akzeptables Vehikel.

2. Zusammensetzung nach Anspruch 1, wobei sowohl R₁ als auch R₂ H sind.

3. Zusammensetzung nach irgendeinem der Ansprüche 1-2, wobei R₃ aus gesättigten oder ungesättigten, linearen, verzweigten oder cyclischen C₂-C₁₂-Kohlenwasserstoffen ausgewählt ist.

4. Zusammensetzung nach irgendeinem der Ansprüche 1 - 3, wobei das 4-substituierte Resorcin-Derivat der allgemeinen Formel I aus der Gruppe ausgewählt ist, bestehend aus 4-Ethylresorcin, 4-Isopropylresorcin, 4-Hexylresorcin, 4-Cyclohexylresorcin und Mischungen davon oder einem kosmetisch akzeptablen Salz davon.

5. Zusammensetzung nach irgendeinem der Ansprüche 1-4, wobei R₃ Hexyl oder Cyclohexyl ist.

6. Zusammensetzung nach irgendeinem der Ansprüche 1-5, wobei R₃ Ethyl ist.

7. Kosmetische Zusammensetzung nach irgendeinem der Ansprüche 1-6, die außerdem ein Hautpflegemittel umfasst, ausgewählt aus der Gruppe, bestehend aus alpha-Hydroxysäuren und Estern, beta-Hydroxysäuren und Estern, Polyhydroxysäuren und Estern, Kojisäure und Estern, Ferulasäure und Ferulat-Derivaten, Vanillinsäure und Estern, Dicarbonsäuren und Estern, Retinol, Retinal, Retinylestern, Hydrochinon, t-Butylhydrochinon, anderen Resorcin-Derivaten, Niacinamid, N-Acetylmethionin, 1-Methylnicotinamidchlorid und Mischungen davon.

8. Lagerstabilisierte kosmetische Zusammensetzung, umfassend:
a. 0,1 bis 2 Gew.-% eines 4-substituierten Resorcin-Derivats der allgemeinen Formel I: 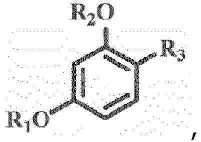
worin R₁ und R₂ jeweils, unabhängig voneinander, aus der Gruppe ausgewählt sind, bestehend aus einem Wasserstoffatom, -CO-R, -COO-R und CONHR, wobei R einen gesättigten oder ungesättigten, linearen, verzweigten oder cyclischen C₁-C₁₈-Kohlenwasserstoff darstellt;
wobei R₃ einen gesättigten oder ungesättigten, linearen, verzweigten oder cyclischen C₁-C₁₈-Kohlenwasserstoff darstellt;
b. 0,0001 Gew.-% bis 5 Gew.-% N,N-Diacetyl-L-Cystin;
c. ein fakultatives Hautpflegemittel, ausgewählt aus der Gruppe, bestehend aus alpha-Hydroxysäuren und Estern, beta-Hydroxysäuren und Estern, Polyhydroxysäuren und Estern, Kojisäure und Estern, Ferulasäure und Ferulat-Derivaten, Vanillinsäure und Estern, Dicarbonsäuren und Estern, Retinol, Retinal, Retinylestern, Hydrochinon, t-Butylhydrochinon, anderen Resorcin-Derivaten, Niacinamid, N-Acetylmethionin, 1-Methylnicotinamidchlorid und Mischungen davon; und
d. ein kosmetisch akzeptables Vehikel;
wobei das Gewichtsverhältnis des N,N'-Diacetyl-L-Cystins zum 4-substituierten Resorcin-Derivat 10.000:1 bis 1:100.000 beträgt, und
wobei das N,N'-Diacetyl-L-Cystin das 4-substituierte Resorcin-Derivat oder das fakultative Hautpflegemittel oder beide in dem Vehikel stabilisiert.

9. Stabilisierte kosmetische Zusammensetzung nach Anspruch 8, wobei das fakultative Hautpflegemittel Niacinamid ist.

10. Verfahren zur Stabilisierung von 4-Ethylrescorcin oder 4-Hexylresorcin in einer Zusammensetzung, umfassend ein kosmetisch akzeptables Vehikel, durch Zugabe von N,N'-Diacetyl-L-Cystin zu der Zusammensetzung in einer ausreichenden Menge, um das 4-Ethylresorcin oder 4-Hexylresorcin zu stabilisieren.

11. Verfahren zur Verwendung der stabilisierten Zusammensetzung nach Anspruch 9 zur topischen kosmetischen Anwendung auf der Haut.

12. Verfahren zur Verwendung von N,N'-Diacetyl-L-Cystin und 4-substituierten Resorcin-Derivaten bei der Herstellung einer topischen kosmetischen Zusammensetzung nach irgendeinem der Ansprüche 1-7 und 8-9.

## Revendications

1. Composition cosmétique topique comprenant :
a. 0,0001 à 10 % en masse de N,N'-diacétyl-L-cystine ; de préférence moins de 1 à 2 % en masse de N,N'-diacétyl-L-cystine ; encore mieux 0,2 % en masse de N,N'-diacétyl-L-cystine ;
b. 0,00001 à 10 % en masse d'un ou plusieurs dérivés de résorcinol 4-substitué de la formule générale I ; de préférence 1-2 % en masse d'un dérivé de résorcinol 4-substitué de la formule générale I ; encore mieux 1 % en masse ou moins d'un dérivé de résorcinol 4-substitué de la formule générale I ; sinon 0,4 % en masse d'un dérivé de résorcinol 4-substitué de la formule générale I : 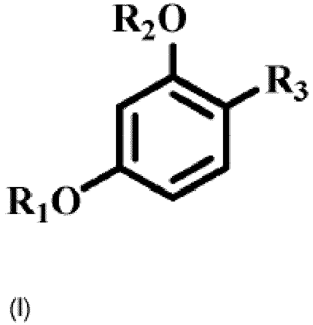
dans laquelle chaque R₁ et R₂ est indépendamment choisi dans le groupe consistant en un atome d'hydrogène, -CO-R, -COO-R, et CONHR ; dans laquelle R représente un hydrocarbure en C₁-C₁₈ saturé ou insaturé, linéaire, ramifié ou cyclique ;
dans laquelle R₃ représente un hydrocarbure en C₁-C₁₈ saturé ou insaturé, linéaire, ramifié ou cyclique ;
et
c. un véhicule cosmétiquement acceptable.

2. Composition selon la revendication 1, dans laquelle à la fois R₁ et R₂ sont H.

3. Composition selon l'une quelconque des revendications 1-2, dans laquelle R₃ est choisi parmi des hydrocarbures en C₂-C₁₂ saturés ou insaturés, linéaires, ramifiés ou cycliques.

4. Composition selon l'une quelconque des revendications 1-3, dans laquelle ledit dérivé de résorcinol 4-substitué de formule générale I est choisi dans le groupe consistant en 4-éthyl résorcinol, 4-isopropyl résorcinol, 4-hexyl résorcinol, 4-cyclohexyl résorcinol, et des mélanges de ceux-ci, ou un sel cosmétiquement acceptable de ceux-ci.

5. Composition selon l'une quelconque des revendications 1-4, dans laquelle R₃ est hexyle ou cyclohexyle.

6. Composition selon l'une quelconque des revendications 1-5, dans laquelle R₃ est éthyle.

7. Composition cosmétique selon l'une quelconque des revendications 1-6, comprenant de plus un agent bénéfique pour la peau choisi dans le groupe consistant en acides et esters alpha-hydroxy, acides et esters bêta-hydroxy, acides et esters polyhydroxy, acides et esters kojiques, acide férulique et dérivés de férulates, acides et esters vanilliques, acides et esters dioiques, rétinol, rétinal, esters rétinyliques, hydroquinone, t-butylhydroquinone, d'autres dérivés de résorcinol, niacinamide, N-acétylméthionine, chlorure de 1-méthylnicotinamide, et mélanges de ceux-ci.

8. Composition cosmétique stabilisée pour le stockage comprenant :
a. 0,1 à 2 % en masse d'un dérivé de résorcinol 4-substitué de la formule générale I : 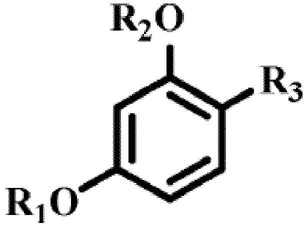
dans laquelle chaque R₁ et R₂ est indépendamment choisi dans le groupe consistant en un atome d'hydrogène, -CO-R, -COO-R, et CONHR ; dans laquelle R représente un hydrocarbure en C₁-C₁₈ saturé ou insaturé, linéaire, ramifié ou cyclique ;
dans laquelle R₃ représente un hydrocarbure en C₁-C₁₈ saturé ou insaturé, linéaire, ramifié ou cyclique ;
b. 0,0001 % en masse à 5 % en masse de N,N'-diacétyl-L-cystine ;
c. un agent bénéfique pour la peau éventuel choisi dans le groupe consistant en acides et esters alpha-hydroxy, acides et esters bêta-hydroxy, acides et esters polyhydroxy, acides et esters kojiques, acide férulique et dérivés de férulates, acides et esters vanilliques, acides et esters dioiques, rétinol, rétinal, esters rétinyliques, hydroquinone, t-butylhydroquinone, d'autres dérivés de résorcinol, niacinamide, N-acétylméthionine, chlorure de 1-méthylnicotinamide, et mélanges de ceux-ci ; et
d. un véhicule cosmétiquement acceptable ;
dans laquelle le rapport en masse de ladite N,N'-diacétyl-L-cystine audit dérivé de résorcinol 4-substitué est de 10 000:1 à 1:100 000 ; et
dans laquelle ladite N,N'-diacétyl-L-cystine stabilise ledit dérivé de résorcinol 4-substitué, ou ledit agent bénéfique pour la peau éventuel, ou les deux, dans ledit véhicule.

9. Composition cosmétique stabilisée selon la revendication 8, dans laquelle ledit agent bénéfique pour la peau éventuel est le niacinamide.

10. Procédé de stabilisation de 4-éthyl résorcinol ou 4-hexyl résorcinol dans une composition comprenant un véhicule cosmétiquement acceptable par addition à la composition de N,N'-diacétyl-L-cystine dans une quantité suffisante pour stabiliser ledit 4-éthyl résorcinol ou 4-hexyl résorcinol.

11. Procédé d'utilisation de la composition stabilisée selon la revendication 9 pour une application cosmétique topique sur la peau.

12. Procédé d'utilisation de N,N'-diacétyl-L-cystine et de dérivés de résorcinol 4-substitué dans la fabrication d'une composition cosmétique topique selon l'une quelconque des revendications 1-7 et 8-9.
